## Europäisches Patentamt
## European Patent Office
## Office européen des brevets

(11) Publication number: **0 190 459**
**B1**

(12) **EUROPEAN PATENT SPECIFICATION**

(45) Date of publication of patent specification: **14.03.90**

(21) Application number: **85116532.4**

(22) Date of filing: **23.12.85**

(51) Int. Cl.⁵: **A 61 K 31/70** // (A61K31/70, 31:045)

(54) **Method of providing total energy requirements to a human who has to be intravenously fed because of illness.**

(30) Priority: **27.12.84 US 686719**
**01.03.85 US 707457**

(43) Date of publication of application:
**13.08.86 Bulletin 86/33**

(45) Publication of the grant of the patent:
**14.03.90 Bulletin 90/11**

(84) Designated Contracting States:
**BE CH DE FR GB LI NL SE**

(56) References cited:
**ROTE LISTE, 1979, Editio Cantor, Aulendorf/Württ., DE; No. 51 174B: "Hamafusal LGX 24/40**

**ROTE LISTE, 1980, Editio Cantor, Aulendorf/Württ., DE.; No. 51 167B: "Caloplasmal 25%/37,5%"; no. 51 171B: "Hochkalorische-Lösung 10%/20%/40%"; no. 51 181B: "Hochkalorische-Lösung LGX 40% mit Elektrolyten"**

The file contains technical information submitted after the application was filed and not included in this specification

(73) Proprietor: **PFRIMMER KABI GMBH & CO. KG**
**Hofmannstrasse 26**
**D-8520 Erlangen (DE)**

(72) Inventor: **Georgieff, Michael, Prof.Dr.**
**Hintere Pfaffenleite 6**
**D-8551 Kunreuth (DE)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90 (DE)**

(56) References cited:
**CHEMICAL ABSTRACTS, vol. 67, no. 7, 14th August 1967, page 2842, no. 30229g, Columbus, Ohio, US; K.G. BAESSLER: "The role of carbohydrates in parenteral feeding", & ANAESTHESIOL. WIEDERBELEBUNG 1966 (6), 20-7**

**K.H. Baessler, Anaestesiol Wiederbelebung 1966(6), 20-27**

Courier Press, Leamington Spa, England.

**Description**

This invention relates to a method of providing total energy requirements of a human who suffers from severe stress or injury, renal disease, liver failure, cancer cachexia or receiving ventilatory support. More specifically, it relates to a carbohydrate mixture e.g., in the form of a solution consisting essentially of glucose and xylitol to cover the energy requirements of a human. The combination of glucose, at a rate no higher than the endogenous glucose production rate between 20 to 200 g/24 hours with xylitol no higher than maximally 210 g/24 hours, will unexpectedly preserve body protein in humans suffering severe illness, trauma, sepsis, organ failure or other injury, as defined herein, when given in above mentioned combination and dosage.

Currently, only solutions containing either glucose, fructose and xylitol, (ROTE LISTE 1979, 51 174 B; ROTE LISTE 1980, 51 167 B; 51 171 B and 51 181 B) or sorbitol and xylitol are available for the treatment of a human. In Chemical Abstracts, Vol. 67, 1967, 30229 g as well as in a former publication of Bässler, K. H. and Reimold, W. V.: Lactatbildung aus Zuckern und Zuckeralkoholen in Erythrocyten. Klin. Wschr. 43: 169—171, 1965 the formation of lactate *in vitro* in erythrocytes during the application of different sugar combinations was tested. An additive effect of lactate formation was found when xylitol and glucose were given together. On page 26 Bässler points out that the various combinations he used in erythrocytes *in vitro* have not yet been tested out clinically. In a study in intensive care patients it now has been found in man, in contrast to the finding of Bässler, in erythrocytes *in vitro* that not only the combination of glucose plus xylitol preserves body protein more efficiently than does an equicaloric infusion of glucose alone, but also whole body glucose turnover, endogenous glucose formation and lactate formation is significantly lower.

The major discovery of this invention is that fructose as well as sorbitol added to xylitol in any form substantially alters the specific useful metabolic effects of xylitol during illness, such as enhancing endogenous fat mobilisation and oxidation, enhancing amino acid exchange from peripheral to visceral tissues and thereby increasing the synthesis of secretory proteins like albumin and transferrin.

Fructose, like xylitol is primarily metabolized in the liver (de Kalbermatten N., Ravussin E., Maeder E. et al, Metabolism 29: 62—67, 1980). In the liver cell fructose is activated to fructose-1-phosphate by fructokinase (Zakim D., Hermann R. H., Am. J. Clin. Nutr. 21: 315—319, 1968), an insulin independent enzyme. The activity of fructokinase though is 5 times higher than all glucouse phosphorylating enzymes in the liver (Zakim D., Hermann R. H., Gordon W. C., Biochem Med. 2: 427—437, 1969), and 4 times higher than the polyol-dehydrogenase activity (Baessler K. H., Stein G., Belzer W., Biochem Z 346: 171—185, 1966) the initial xylitol degradating enzyme. Fructose-1-phosphate is cleaved by a specific aldolase to two trioses (Zakim D., Hermann R. H., Gordon W. C., Biochem Med. 2: 427—437, 1969). In contrast to the aldolase which cleaves fructose-1,6-diphosphate from glucose and xylitol metabolsim, the fructose-1-phosphate specific aldolase has a very high activity and is not a rate limiting enzyme (Zakim D., Hermann R. H., Gordon W. C., Biochem Med. 2: 427—437, 1969). The rapid initial phosphorylation of fructose and the unlimited cleavage to trioses allows dietary fructose to be metabolized in the Emden-Meyerhof-Pathway in the liver at a far greater rate than glucose and xylitol (Baessler K. H., Stein G., Belzer W., Biochem Z 346: 171—185, 1966). The high turnover rate in the glycolytic pathway during frucotse administration therefore increases lactate formation, hepatic fat synthesis. Compared to other carbohydrates like glucose and xylitol, fructose underlies an enhanced conversion to fat in the liver and by increasing the amount of endogenous free fatty acid reesterified to triglycerides in the liver, fructose leads to damage of the liver during total parenteral nutrition (intravenous feeding); (Forster H., Internist 19: 2—19, 1977). All these metabolic effects can be observed even at low infusion rates ranging from 20—100 g.

Sorbitol is a polyalcohol like xylitol. Sorbitol is also primarily metabolized in the liver (Baessler K. H., Pharm. Therap. Dent. 3: 85—93, 1978) and there dehydrogenated by the same polyol-dehydrogenase as xylitol (Froesch E. R., Zapf J., Keller U., et al, Europ. J. Clin. Invest. 2: 627—633, 1978) to fructose. From that step on sorbitol is metabolized as is fructose, and can cause the same metabolic side effects even at low infusion rates.

Whenever either fructose or sorbitol are added to a solution containing xylitol, the principal metabolic effects of xylitol, moderation of elevated blood glucose and insulin levels, reduction of gluconeogenesis, increase in protein synthesis and enhancement of endogenous as well as exogenous fat oxidation, are attenuated.

Glucose, given above the endogenous production rate of approximately 200 g/24 hours is associated with an increase of blood glucose and insulin levels (Wolfe R. R., Allsop J. R., Burke J. F., Metabolism 28: 210—220, 1979). After injury, glucose given above the endogenous production rate stimulates hepatic lipid synthesis (Wolfe R. R., O'Donell T. F., Stone M. D., Metabolism 29: 892—900, 1980), and reduces endogenous amino acid flux from peripheral to visceral organs (Moldawer L. L., O'Keefa S. J. D., Bothe A., et al, Metabolism 29: 173—180, 1980), thus reducing overall protein synthesis due to restricted amino acid availability. The percentage of infused glucose oxidized, declines when given above the endogenous production rate (Wolfe R. R., Allsop J. R., Burke J. F., Metabolism 28: 210—220, 1979), thus contributing less effectively to energy expenditure. In order not to attenuate the metabolic response to severe illness during intravenous feeding with glucose, which is primarily characterised by a mobilisation of the body's own reserves in order to optimize protein synthesis and host defence, glucose should only be given in a dosage under the endogenous production rate ranging from 20—200 g/24 hours.

Accordingly it is a goal of this invention to feed the critically ill intravenously so as to moderate blood glucose and insulin elevations and to attenuate the loss of lean body mass by reducing gluconeogenesis and increasing protein synthesis. Current state of the art tries to maintain body protein by stimulating endogenous insulin secretion, or giving it exogenously (Hinton P., Allison S. P., Littlejohn S., Lancet 2: 767—769, 1971), and thereby try to reduce muscle protein breakdown (Woolfson A. M. J., Hertley R. V., Allison S. P., New Engl. J. Med. 300: 14—17, 1979). During the hypoinsulinemic state of trauma, xylitol is oxidized at a significantly higher rate than glucose without extensive hyperglycemia (de Kalbermatten N., Ravussin E., Maeder E., et al, Metabolism 29: 62—67, 1980). Xylitol enters the pentose phosphate shunt directly and does not require insulin (Hollman V. S., Reinauer H., Z. Ernaehrungswiss. 11: 1—7, 1971).

Depending on the severity of injury, the maximal disposal rate of xylitol increases from 0.37 g/kg BWxh in normal to 0.76 g/kg Bwxh after injury (Ackerman R. H., Infusionstherapie 7: 113—115, 1980). In contrast to xylitol, maximal glucose disposal rate after injury is reduced by approximately 36%, even supraphysiologic insulin concentrations are not capable of increasing the limit after injury (Blach P. R. Brooks D. C., Bessey P. Q., et al., Ann. Surg. 196: 420—433, 1982). Therefore any glucose infusion above the endogenous production rate of 200 g during illness will be associated with an increase of blood glucose and insulin levels, causing fatty liver, reduced protein synthesis and inefficient protein preservation. Unlike glucose, intravenously administered xylitol is primarily metabolized in the liver and there converted to glucose independent of insulin, most importantly, xylitol generates the intermediates of glucose metabolism important for amino acid and fat utilisation without having to generate glucose (Pellaton M., Acheson K., Maeder E., et al, JPEN 2: 627—633, 1978).

The invention described herein is a novel parenteral nutrition solution made from xylitol and glucose as the only carbohydrates, and a method of treatment employing it for use in a human suffering severe stress or injury or significant renal or hepatic disease to reduce nitrogen wasting and its accelerated gluconeogenesis. The novel solution of the invention comprises an aqueous xylitol and glucose solution, each no more than 200 g each and no less than 20 g each, suitable for either peripheral (if given at low concentrations) or central venous administration (infusion).

Another aspect of the present invention comprises a method of treatment for a critically ill patient suitable for intraveous purposes to cover the energy expenditure, reduce gluconeogenesis and protein wasting as well as promote endogenous fat oxidation, ketogenesis and amino acid availability for hepatic secretory protein synthesis, leucocytosis and for wound healing.

The invention described herein is based partly upon the recognition that reducing the wasting of tissue protein and gluconeogenesis are critical to effective and successful recovery. In contrast to current state of the art of treating a patient, where protein preservation is tried to be achieved by mainly reducing protein catabolism, this new method preserves body protein by primarily increasing protein syntehsis, which is a far more effective way in retaining body protein content. Accordingly, an increased fatty acid oxidation will reduce the obligatory need to catabolize tissue protein for energy and therefore provide more precursors for wound healing, leucocytosis and hepatic secretory protein synthesis, essential for recovery.

Types of humans being beneficially treated herein are for example those having a body nitrogen loss greater than 4 g/day or blood glucose concentration greater than 120 mg/ml.

It should be understood that other nutritional substances may be administered so long as sorbitol or fructose are not included and so long as these substances do not interfere with the usefulness of the composition of the invention.

The following examples further describe the invention.

A sterile, nonpyrogenic, stable solution suitable for intravenously infusion into a peripheral or central vain of critically ill traumatized patients is prepared from pure anhydrous glucose and anhydrous xylitol, which are dissolved in distilled water in the following concentrations:

| Examples | Glucose g/l | Xylitol g/l |
|---|---|---|
| a | 20 | 100 |
| b | 50 | 100 |
| c | 100 | 100 |
| d | 150 | 150 |
| e | 150 | 200 |
| f | 200 | 50 |
| g | 100 | 20 |

Sterilization is carried out in a conventional manner. In the foregoing formula, the ratio of glucose to xylitol is variable, but the concentration of each energy source should be no higher than maximally 200 g each.

As used in this application, glucose is used in its ordinary sense as the active D-glucose.

As supporting evidence for the uniqueness of a glucose/xylitol mixture as an energy source in the preservation of body protein compared to the use of either glucose or xylitol alone in parenteral feeding serve the following clinical tests:

Patients with severe trauma received either 210 g/day of xylitol (Group I), 210 g/day of glucose (Group II) or 210 g/day of a xylitol/glucose mixture (1:1) (Group III) as energy source. All three groups received 2 g/kg BW day amino acids. The infusion rates are related to a body weight of 70 kg. Oral nutrient intake was contraindicated in all patients.

Nitrogen balance

|  | Day 1 | Day 2 | Day 3 | Day 4 |  |
| --- | --- | --- | --- | --- | --- |
| Group (I), n=3 | −9.1±3.1 | −8.7±2.4 | −9.5±1.9 | −10.7±2.9 | (Xylitol) |
| Group (II), n=3 | −12.3±2.7 | −10.6±2.8 | −13.2+3.1 | −15.6+3.5 | (Glucose) |
| Group (III), n=3 | −5.2±2.2 | −5.5±2.1 | −6.3±2.5 | −5.9±3.2 | (Glucose+Xylitol) |

**Claims**

1. A sterile aqueous preparation as a solution for providing an energy source for intravenous feeding comprising as carbohydrates glucose and xylitol only, in an amount of 20 to 200 g each.

2. Use of the amount of glucose and xylitol as claimed in claim 1 per liter for the manufacture of an aqueous preparation for providing energy to a human in need thereof.

3. Use according to claim 2 for a preparation for the treatment of a human suffering severe trauma, injury, burn or infection in which body nitrogen loss is greater than 4 g/day or blood glucose concentration is greater than 120 mg/ml.

4. Use according to claim 2 for a preparation for the treatment of renal disease.

5. Use according to claim 2 for a preparation for the treatment of hepatic disease.

6. Use according to claim 2 for a preparation for the treatment of cancer cachexia.

**Patentansprüche**

1. Sterile wäßrige Zubereitung als Lösung zur Zurverfügungstellung einer Energiequelle zur intravenösen Ernährung, enthaltend als Kohlenhydrate nur Glucose und Xylit in einer Menge von jeweils 20 bis 200 g.

2. Verwendung der in Anspruch 1 angegebenen Menge an Glucose und Xylit pro Liter zur Herstellung einer wäßrigen Lösung zur Zurverfügungstellung von Energie für einen Menschen, der dieser bedarf.

3. Verwendung nach Anspruch 2 für eine Zubereitung zur Behandlung von Menschen, die an schwerem Trauma, Verletzungen, Verbrennungen oder Infektionen leiden, bei denen der Stickstoffverlust des Körpers größer als 4 g pro Tag ist oder die Blutglucosekonzentration größer als 120 mg/ml ist.

4. Verwendung nach Anspruch 2 für eine Zubereitung zur Behandlung von Nierenerkrankungen.

5. Verwendung nach Anspruch 2 für eine Zubereitung zur Behandlung von Lebererkrankungen.

6. Verwendung nach Anspruch 2 für eine Zubereitung zur Behandlung von Krebskachexie.

**Revendications**

1. Préparation aqueuse stérile sous forme de solution pour fournir une source d'énergie pour l'alimentation intraveineuse contenant comme glucides du glucose et du xylitol uniquement en une quantité de 20 à 200 g chaque.

2. Utilisation de la quantité de glucose et de xylitol par litre selon la revendication 1 pour l'obtention d'une préparation aqueuse pour un apport d'énergie à un patient en ayant besoin.

3. Utilisation selon la revendication 2 pour une préparation pour le traitement d'un patient souffrant d'un traumatisme, d'une lésion, d'une brûlure ou d'une infection sévère chez lequel les pertes azotées sont supérieures à 4 g/jour et la glycémie est supérieure à 120 mg/ml.

4. Utilisation selon la revendication 2 pour une préparation pour le traitement d'une maladie rénale.

5. Utilisation selon la revendication 2 pour une préparation pour le traitement d'une maladie hépatique.

6. Utilisation selon la revendication 2 pour une préparation pour le traitement de la cachexie cancéreuse.